# EUROPEAN PATENT SPECIFICATION

(11) **EP 1 753 803 B1**
(45) Date of publication and mention of the grant of the patent: **08.04.2009**
(21) Application number: 05751092.7
(22) Date of filing: 09.06.2005
(51) Int. Cl.: C08G 69/10, A61K 47/34, A61K 47/48, A61K 9/20

(54) **POLYPEPTIDES WITH THE CAPACITY TO ENTRAP DRUGS AND RELEASE THEM IN A CONTROLLED WAY**
POLYPEPTIDE MIT FÄHIGKEIT ZUM EINFANGEN UND KONTROLLIERTEN FREISETZEN VON WIRKSTOFFEN
POLYPEPTIDES CAPABLES D'ENCAPSULER DES SUBSTANCES MEDICAMENTEUSES ET DE LES LIBERER DE MANIERE CONTROLEE

(30) Priority: 10.06.2004 US 578929 P
(43) Date of publication of application: 21.02.2007
(73) Proprietor: Farmhispania S.A., 08008 Barcelona (ES)
(72) Inventor: LLEDE, Ernest, Giralt, E-08960 Sant Just Desvern (ES); POYATOS, Pau, Cid, E-08225 Terrassa (ES); ANGLADA, Frances Rabanal, 08859 Begues Barcelona (ES); PORRAS, Jose Pastor, 08750 Molins de Rei Barcelona (ES); GULIN, Oscar Pena, 08027 Barcelona (ES)
(74) Representative: Campbell, Neil Boyd
(86) International application number: PCT/IB2005/001613
(87) International publication number: WO 2005/121215

(56) References cited:
- EP-A- 1 285 942
- WO-A-00/52078
- US-A1- 2003 104 989

## Description

### FIELD OF THE INVENTION

The present invention relates to new crosslinked peptide polymers or polypeptides with the capacity to entrap pharmaceutically active components or material and, subsequently, release them in a controlled way in a physiological medium.

### BACKGROUND OF THE INVENTION

In the field of drug delivery two of the most important - and often associated - pharmacological problems concern the difficulty of maintaining the concentration of the active component at therapeutic levels for prolonged periods, and enabling this active component to reach its therapeutic target. These problems particularly affect drugs with a short half-life, such as peptides or proteins, where effective treatment requires frequent parenteral administration, with all its associated adverse effects for patients.

Technical solutions have been developed to ease these effects, and mainly entail the entrapment of the drug or bioactive molecules in polymeric matrices able to release them in a controlled manner and, if possible, only when they reach the therapeutic target.

To date, some of the matrices most widely used for this purpose have been PLGA polymer compositions, these being co-polymers of lactic acid and glycolic acid. However, the drug release kinetics of many of these matrices is difficult to control, and is often not of the desired zero order, as the process by which the matrix microspheres are formed causes the drug to accumulate at the surface and induces a massive release of the drug in the initial stages of release. Furthermore, degradation of PLGA matrices through hydrolysis of their ester groups takes place throughout the microspheres, not only at their surface; this makes the microspheres porous and, consequently, the drug is released at a faster rate.

International patent application WO-A-00/52078 describes amino acid polymers that are able to be crosslinked under certain conditions, the resulting matrices being able to entrap drugs in their structure.

The cited international patent application describes polypeptides formed by between one and more than seven amino acids, selected from among glutamic acid (Glu), lysine (Lys), phenylalanine (Phe), proline (Pro), tryptophan (Trp), tyrosine (Tyr) and cysteine (Cys). It is reported that these polypeptides can reorganize themselves into higher-order structures, sometimes forming hydrophobic domains which are useful for protecting sensitive compounds from chemical and enzymatic proteolysis, and that under certain conditions they are able to release these compounds in a controlled way.

A structural characteristic of the polypeptides described in WO-A-00/52078 is that the polymers obtained through the formation of peptide bonds between one or more of the above-mentioned amino acids do not contain additional functional modifications in the monomeric units of their peptide chain, thus limiting their ability to be crosslinked and, consequently, to form polymer matrices with the capacity to entrap drugs.

Chemical Abstracts 2001:197706 includes an abstract published in Abstr. Pap. -Am. Chem. Soc (2001), 221^{st}. The original publication is a conference abstract, not a detailed paper, and reports that protein microspheres may be used as drug release systems. According to the abstract the microspheres are obtained by applying ultrasound to proteins which contain cysteine residues able to form disulfide bonds, thus producing the crosslinking which leads to the formation of microspheres able to entrap drugs. The cited publication, however, does not describe the types of protein used, or the technical details required to reproduce the study in the laboratory.

Thus, it can be seen that the state of the art regarding the use of polypeptides to form matrices with the capacity to entrap drugs and release them in a controlled way is far from developed. Indeed, it could be said to be taking its first tentative steps. Therefore, there is a need to find new, specific, technical solutions in this field that are effective in developing new drug delivery systems, especially for those active components which, as was pointed out above, have a short half-life and frequently require parenteral administration.

### SUMMARY OF THE INVENTION

One object of the present invention is to develop new polypeptides which can be crosslinked in aqueous medium and which entrap suspended or dissolved active pharmaceutical components or materials, forming polymer matrices with the capacity to release pharmaceutical components in a controlled way within a physiological medium.

Another object of the invention is to develop a procedure for preparing the new polypeptides.

Yet another object of the present invention is to produce polymer matrices able to contain drugs, obtained when the polypeptides are crosslinked in the presence of at least one drug.

It is also an object of the invention to develop a procedure for producing the above-mentioned polymer matrices able to contain drugs.

It is a further object of the invention to develop pharmaceutical compositions containing the polymer matrices, obtained from the new polypeptides mentioned above, and which include one or more active pharmaceutical components.

A still further object of the invention is to use the new polypeptides to produce matrices able to release drugs in a controlled way.

### BRIEF DESCRIPTION OF THE FIGURES

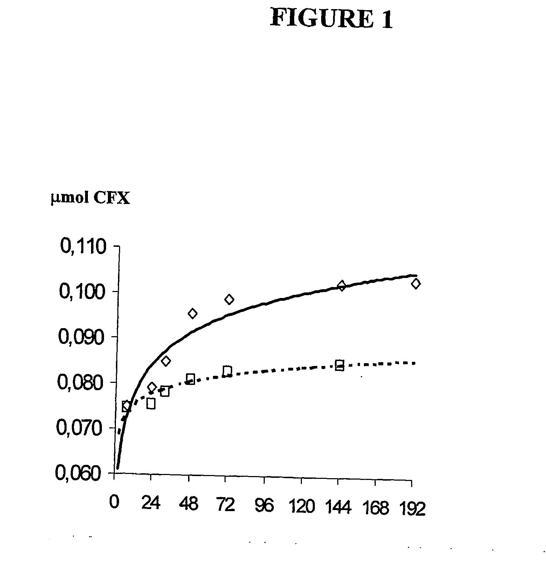

Figure 1 is a graph showing the release kinetics of ciprofloxacin (µmol of ciprofloxacin against time) when it was contained within one of the polymeric matrices of the present invention. The graph shows a comparison between the sample subjected to enzymatic proteolysis (M1) and a control without enzyme (B).

### DESCRIPTION OF THE INVENTION

The expressions "polypeptides", "peptide polymer" and "peptide polymers" used herein refer to polymer structures formed by monomeric units which result from peptide bond formation of natural or non-natural amino acids and/or their derivatives; these amino acids may be protected.

Furthermore, when reference is made to protective groups or ways of protecting functional groups "as described by Green", this refers to the well-known book on the subject, namely, Green et al. "Protective Groups in Organic Synthesis" Third Ed. John Wiley & Sons Inc. 1999 (ISBN 0-471-16019-9).

The present invention has, for the first time, resulted in a new kind of polypeptide which can be crosslinked under mild conditions, and which produces polymer matrices which can entrap drugs and release them in a controlled way in a physiological medium.

The polypeptides of the present invention comprise polymers having between about 1% and about 75% of their monomeric units with one of the following formulas (I), (II) or (III): 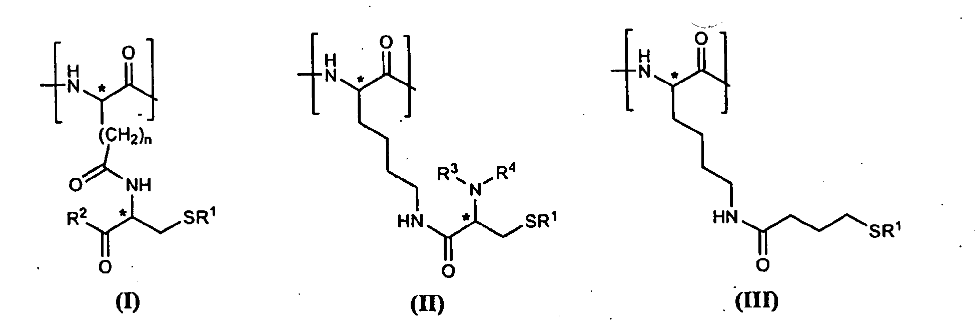 wherein:
- n can be 1 or 2;
- R¹ can be H or a thiol protective group, such as those described in Green;
- R² can be a hydroxyl group (OH), a carboxylic acid protective group, such as those described by Green, or a -NR⁵R⁶ group in which R⁵ and R⁶ may be either H or a C₁-C₇ alkyl group, linear or branched;
- R³ and R⁴ may be H, a C₁-C₇ alkyl group, linear or branched, or an amine protective group, such as those described in Green.
   The chiral centres marked witch an asterisk may have an *R, S* or *RS* configuration.

In the preferred embodiment between about 5% and about 30% of the monomeric units of the polypeptides of the present invention have one of the formulas (I), (II) or (III).

Also in the preferred embodiment the polypeptides of the present invention have a molecular weight of between about 5,000 and about 200,000, more preferably between 10,000 and 100,000.

In a preferred embodiment at least about 50% of the monomeric units of the polypeptides of the present invention have the formula (IV) or (V) 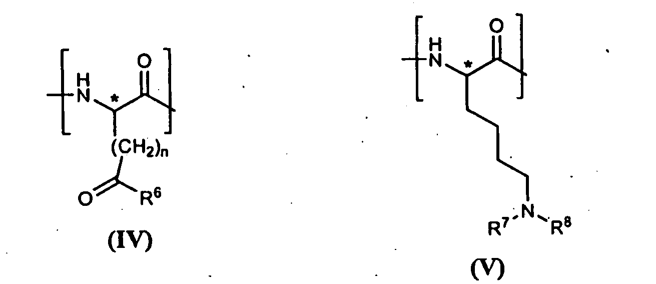 wherein:
- n can be 1 or 2;
- R⁶ can be a hydroxyl group (OH), a carboxylic acid protective group, such as those described by Green, a -NR³R⁴ group in which R³ and R⁴ are as stated above, or a residue of the formula 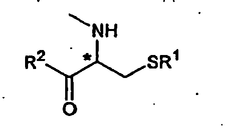 where R¹ and R² are as stated above;
- R⁷ and R⁸ can be H, a C₁-C₇ alkyl group, linear or branched, or a primary amine protective group, such as those described by Green and in which case either R⁷ or R⁸ is H, or a residue of the formula 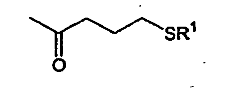 in which case either R⁷ or R⁸ is H and R¹ is as stated above, or a residue of the formula 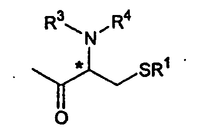 in which case either R⁷ or R⁸ is H and R¹, R³ and R⁴ are as stated above.
The chiral centres marked with an asterisk may have an *R*, *S* or *RS* configuration.

In the above-mentioned preferred embodiment the polypeptides may contain other monomeric units selected from among other natural or non-natural amino acids and their protected derivatives.

The preferred polypeptides are those which have one or more of the following features:
- at least about 80% of the monomeric units have the formula (IV) or (V);
- the proportion of monomeric units containing SR¹ groups is between about 5% and about 30%;
- the monomeric units may be selected from those of formula (IV), those of formula (V), and other amino acid residues, whether of L or D configuration, the preferred forms being L-alanine and D-alanine;
- there are also monomeric units selected from those with formula (IV) or (V) in which R¹ is H, that is, they contain free thiol groups;
- there are also monomeric units selected from those with formula (IV) in which R⁶ is a hydroxyl group (OH) or a cysteine residue, and from those with formula (V) in which R⁷ and R⁸ are H or R⁷ is H and R⁸ is a residue of either cysteine or 4-mercaptobutyroyl.

Especially preferred are the following groups of polypeptides:

Group A: polymers which contain monomeric units of residues of L-glutamic acid and L-glutamic acid (L-cysteine) with the formulas

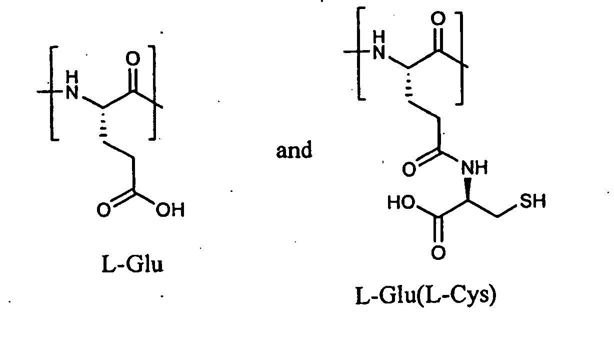

Group B: polymers like those of group A but which also contain monomeric units of L-alanine residues.

Group C: polymers which contain monomeric units of residues of L-lysine and N'-(4-mercaptobutyroyl)-L-lysine with the formulas

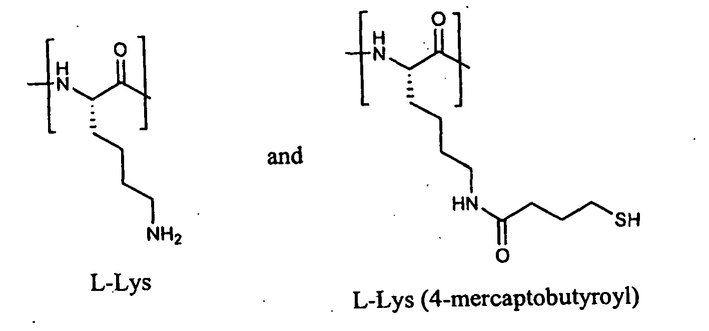

-Group D: polymers like those of group C but which also contain monomeric units of L-alanine residues.

In another especially preferred embodiment, between about 5% and about 30% of the monomeric units of the polymers of groups A, B, C and D contain free thiol groups (SH).

The polypeptides of the present invention are prepared by means of amidation reactions, starting from precursor peptide polymers in which at least about 50% of the monomeric units have the formula (VI) or (VII) 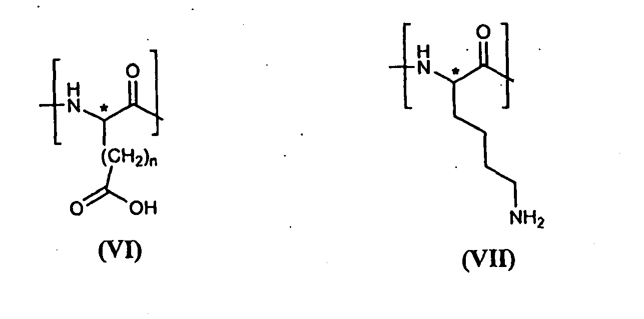 or their precursors in which either the carboxylic acid group or the free primary amine group are protected by protective groups such as those described by Green. This means that:
- the precursor polypeptide containing monomeric units of formula (VI) reacts with cysteine or its protected derivatives with the formula 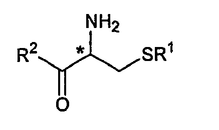 in which R¹ and R² are as stated above; or alternatively,
- the precursor polypeptide containing monomeric units of formula (VH) reacts with cysteine or its protected derivatives with the formula 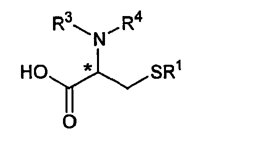 or with 4-mercaptobutyric acid or its activated derivatives.

If desired, the protective groups can then be completely or partially removed from those functional groups which remain protected.

The precursor polypeptides can be prepared using conventional procedures of peptide synthesis starting from glutamic acid, aspartic acid or lysine, in its L, D or LD forms, these being the amino acids which correspond to the monomeric units with formula (VI) or (VII); other natural or non-natural amino acids may also be used, preferably alanine, in any one of its L, D or LD forms, or its protected derivatives. In all cases, at least about 50% - and preferably at least about 80% - of the monomeric units must have the formula (VI) or (VII).

Some of these precursor polypeptides, such as polymers of polyglutamic acid (polyGlu) or polylysine (polyLys) of various molecular weights, are known, and are commercially available.

One suitable method for carrying out the polymerization reactions is that involving the formation of N-carboxyanhydride (NCA) activated intermediates, as described, for example, in Katakai et al. J. Org. Chem. 1985, 50, pp 715-716, followed by polymerization of the activated NCA amino acids as described, for example, in Blout et al. J. Am. Chem. Soc. 1956, 78, pp 941-946.

In order to prepare precursor polypeptides from amino acids containing an additional carboxylic group, glutamic acid and aspartic acid, it is preferable to use those in which the additional carboxylic group is protected, for example, in its benzyl ester form; the protection of the carboxylic groups can then be completely or partially removed after polymerization.

The amidation reaction of precursor polymers containing carboxylic groups - monomeric units of formula (VI) - with cysteine or its protected derivatives is performed using standard techniques of amide bond formation. Thus, for example, after completely or partially removing the protection from the carboxylic groups, the free carboxylic groups are then activated using any of the usual techniques of peptide synthesis, for example, by addition of water-soluble carbodiimide: the reaction with cysteine can then be performed.

For precursor polymers containing primary amine groups - monomeric units of formula (VII) - the amidation reaction involves the acylation of these amino groups using conventional methods. For example, the amine groups can be acylated with 4-butyrolactone or with activated derivatives (which may have a protected thiol group) of 4-mercaptobutyric acid. They can also be acylated with cysteine derivatives activated in the carboxylic group, which may have a protected amine group.

Once the functionalized polypeptides have been obtained the protective groups can be removed from any thiol groups which were protected, using the deprotection techniques described by Green.

The degree of functionalization of the polymer is the percentage of monomeric units containing free thiol groups, and can be determined using the Ellman quantitative test, as described in Ellman Arch. Biochem. Biophys. 1958, 74, pp 443-458. The degree of functionalization can vary freely and depends on the amount of monomeric units of formulas (VI) and (VII) in the precursor polymer, and on the stoichiometry of the subsequent amidation reaction with the thiolated compounds. As has already been pointed out, between about 1% and about 50% of the monomeric units of the polymers of the present invention are thiolated, and preferably between about 5% and about 30%.

The polypeptides of the present invention can be crosslinked in aqueous medium, at a pH equal to or greater than 6, because the thiol groups are oxidized with oxygen from the air and form disulfide bonds. The crosslinking leads to the formation of polymer matrices with the capacity to entrap drugs that are suspended or dissolved in the aqueous medium.

These polymer matrices containing drugs can be isolated from the aqueous medium using conventional techniques, for example, through concentration with dialysis membranes and subsequent centrifugation.

One procedure for preparing the drug-containing polymer matrices of the present invention is to oxidize the polypeptides of the present invention in alkaline aqueous medium, in the presence of a drug, and separate the resulting product.

One of the main advantages of this technique, compared with conventional methods which rely on the formation of microspheres with PLGA polymers, is that the whole process of drug entrapment is carried out without the need for organic solvents that interact with the drug, and which are difficult to eliminate afterwards. Moreover, if the drug is protein-based the solvent may cause it to be denatured, and thus it loses its activity.

When the polymer matrices of the present invention are introduced into the physiological medium they release the drug in a controlled way because their peptide nature enables progressive degradation under the action of enzymes present in the medium. If suitable monomeric units are selected, for example, with respect to the number of monomers originating from D-amino acids, the drug release can be modulated as desired by using matrices with varying speeds of enzymatic proteolysis.

In the matrices of the present invention the entrapped drug is distributed more or less uniformly and, furthermore, it is released in a regular way because the enzymatic degradation of the polymer matrix takes place at the surface; this means that the drug release speed is constant. These features also constitute significant advantages over conventional PLGA microspheres which, as pointed out above, rarely achieve zero- order kinetics, owing to accumulation of the drug at their surface and their irregular degradation.

A further advantage of the polymer matrices of the present invention is that, due to their peptide nature, they are biodegradable and well-tolerated by the human body.

In principle, the polypeptides of the present invention may be used to obtain polymer matrices with the capacity to release various kinds of drugs in a controlled way; they are therefore suitable for use in pharmaceutical compositions aimed at any kind of treatment, especially prolonged treatment.

It should be pointed out, however, that the polymer matrices of the present invention are especially suitable for delivering active compounds which, when administered in conventional pharmaceutical forms, require frequent parenteral administration, with all its associated adverse effects for patients.

The polymer matrices of the present invention are also especially suitable for active components or materials with high therapeutic activity and which present delivery problems owing to their high degradability in the organism, as is the case of peptides and proteins.

For purely illustrative purposes, the following can be mentioned as examples of these kinds of active components or materials: the peptide T-20, currently being used with patients infected with HIV-1 (Nature Medicine, 1998, 4, 1302-1307; Proc. Natl. Acad. Sci., 1994, 91, 9770-9774) and which has to be administered in very high and frequent doses; gonadotropin-releasing hormone (GnRH) analogues such as leuprolide, goserelin, nafarelin or triptorelin, commonly used in the treatment of certain kinds of cancer, mainly prostate cancer; interferon and other interleucines with multiple medical applications, among others, as cancer treatments; follicle-stimulating hormone (FSH) and luteinizing hormone (LH) used in fertility treatments.

None of the above prevents the polymer matrices of the present invention from also being suitable for containing other drugs of important therapeutic interest such as, to cite two widely-used examples, the anti-cancer drugs taxol and doxorrubicin. Studies have shown that the therapeutic index of paclitaxel (taxol) is improved when it is conjugated with polyglutamic acid (Li, Chun. Advanced Drug Delivery Reviews. 2002, 54(5), 695-713; Auzenne et al. Clinical Cancer Research. 2002, 8(2), 573-581.

Pharmaceutical compositions useful in the treatment of disease can be obtained with the drug-containing polymer matrices of the present invention. The pharmaceutical compositions thus obtained may be designed for any administration route (oral, parenteral, subcutaneous, transdermic, transmucosal, etc.) and may contain the excipients and adjuvants commonly used in pharmaceutical technology.

In the preferred embodiment, the pharmaceutical compositions of the present invention are those designed for parenteral administration.

### EXAMPLES

### Example 1 Preparation of a glutamic acid polymer functionalized with cysteine, polyGlu(Cys)_{0,21}-OH

### (a) Preparation of the precursor polymer, polyGlu(OH)

5 g of γ-benzyloxyglutamic acid (H-Glu(OBz)-OH, 21 mmol) is suspended in 60 mL of dry tetrahydrofuran, which has been previously eluted through an alumina column and distilled over sodium, and then heated to 50° C under anhydrous conditions. Triphosgene (98%, 3.1 g, 0.5 eq., 10.4 mmol) is then added. The reaction mixture is shaken for at least 3 h until the initial suspension becomes a clear solution. Finally, it is allowed to cool and is rotatory evaporated to 15-20 mL. The solution is diluted with 45 mL of distilled AcOEt and filtered. 300 mL of distilled hexane are then added to the filtrate and the mixture is cooled in a bath of acetone and dry ice to -78 ° C. The resulting precipitate is filtered on a filter plate, dissolved in 30 mL of distilled AcOEt and precipitated with 300 mL of distilled hexane.

The N-carboxyanhydride obtained (2.25 g, 8.5 x 10⁻³ mol) is dissolved in dioxane (30 mL, filtered through an alumina column, distilled over sodium and stored over a molecular sieve). Et₂NH is then added (9 µl, 85 µmol 99%) and the mixture is left, without shaking, for 5 days in dry air. An aqueous solution of HCl (200 mL, 6.5 mM) is then added and the mixture is left at room temperature for 4 h. The resulting precipitate is filtered and washed with abundant water until a neutral pH is reached. A total of 1.3 g of the protected polymer polyGlu(OBz) (65% yield) is obtained.

A portion of the protected polymer (250 mg) obtained is suspended in 1 mL of AcOH and shaken for 2 h under anhydrous conditions. Then, 10 mL of an HBr/AcOH (3:1) mixture is then added followed by shaking for 15 h. The product is then precipitated with 200 mL of Et₂O and filtered using a filter plate. The polyGlu(OH) obtained is stored in a dessicator under KOH. Benzyl removal is assessed by UV-Vis at 254 nm. Yield: 90 mg, 70%. The mean molecular weight of the obtained polymer is 25 kDa.

### (b) Preparation of polyGlu(Cys)_{0,21}-OH

Polyglutamic acid (polyGlu(OH)) ( SIGMA, 17 kDa, 20.2 mg, 132 µmol of COOH groups) is treated with (1-ethyl-3-(3'-dimethylaminopropyl) carbodiimide HCl (NOVABIOCHEM, 26.3 mg, 0.137 µmool) at pH of 5 (MES buffer, 2-(N-morpholino)ethansulphonic acid, 100 mM, 150 µl). 30 min later, cysteine (SIGMA, 10 eq., 208.1 mg, 1.32 mmol) is added, the solution is increased to a volume of 1150 µl with additional MES buffer and the pH is readjusted to around 6 with NaOH 10 N (1-2 µL). The resulting solution is shaken at room temperature for 20 h. The disulfide bridges are reduced with an excess of sodium borohydride (NaBH₄, approximately 15 mg). The polymer is purified by dialysis in the presence of aqueous HCl (1 mM, 1L) for 8 h, using MWCO 1000 Da membranes (CELLU-SEP). Dialysis is performed in triplicate and the polymer obtained by lyophilization.

### Example 2 Preparation of a lysine polymer functionalized with 4-mercaptobutyroyl

4-butyrothiolactone (7 µL, 0.08 mmol) is added to a buffered water solution (1 mL, borate, pH 9) of polyLys (20 mg, 0.16 mmol NH₂, molecular weight 26 kDa, SIGMA) in an argon atmosphere with vigorous magnetic shaking. After 24 h, maintaining the pH at 9 by the addition of NaOH 1M, the solution is treated with an excess of NaBH₄ (approximately 15 mg). The thiolated polymer is diluted with Milli-Q water (4 mL) and purified by dialysis in the presence of aqueous HCl (1 mM, 1L) for 8 h (using MWCO 1000 Da membranes, CELLU-SEP). Dialysis is performed in triplicate. Finally, the aqueous solution is lyophilized in order to obtain polyLys(CO(CH₂)₃-SH)_{0,12}.

### Example 3 Preparation of an alanine and glutamic acid co-polymer, functionalized with cysteine, polyAla-polyGlu(OBz)ₓ₍Cys)_{y}(OH)_{z}

### (a) Preparation of the precursor co-polymer, polyAla-polyGlu(OBz)

Alanine (H-Ala-OH) (4 g, 43.4 mmol) is suspended in tetrahydrofuran (100 mL, eluted through an alumina column and distilled over sodium) and heated to 50° C under anhydrous conditions (CaCl₂ tube), before adding triphosgene (6.4 g, 21.7 mmol, 98%). The reaction mixture is shaken for 3-4 h. It is then allowed to cool and is concentrated by rotatory evaporation to 15-20 mL. The solution is diluted with 35 mL of AcOEt and filtered. Hexane (250 mL) is added to the solution which is cooled in a bath of acetone and dry ice to -78° C. The precipitate is filtered on a filter plate and dissolved in 30 mL of AcOEt, and then precipitated with 250 mL of hexane. The product obtained is the N-carboxyanhydride of the alanine, NCA-Ala, which is stored in a dessicator over P₂O₅. Yield: 3.0 g, 60%.

The NCA-Ala obtained in the previous stage (50 mg, 4.35 x 10⁻⁴ mol) is dissolved in 1,4-dioxane (1.4 mL, filtered through an alumina column, distilled over sodium and stored over a molecular sieve). Poly-γ-benzylglutamic acid (120 mg 1.739 x 10⁻⁶ mol, SIGMA, molecular weight 69 kDa,) is then added and dissolved completely with magnetic stirring. The mixture is left at room temperature, without stirring and in dry air, for 4 days. The gel obtained is then poured over an aqueous solution of HCl (200 mL, 6.5 mM). The resulting precipitate is filtered and washed with abundant water until the wash water has a neutral pH. 106 mg (70% yield) of the benzylated copolymer (Ala)₁₇₈-(Glu(OBzl))₃₁₅ is obtained, and this is identified by NMR-¹H, IR and amino acid analysis.

### (b) Preparation of polyAla-polyGlu(OBz)ₓ(Cys)_{y}(OH)_{z}

The benzylated copolymer obtained in the previous stage is then partially debenzylated using the methodology described in M. Bodanzsky & A. Bodanzsky The Practice of Peptide Synthesis. Springer Verlag Ed. Berlin 1984, pp 177-178. The removal of protection from the benzyl group may be total or partial, depending on the conditions and stoichiometry of the reaction.

Using the functionalization method with cysteine described in stage b) of example 1, the desired proportion of carboxylic groups can then be functionalized simply by making any necessary adjustments to the reaction stoichiometry.

Thus, varying degrees of copolymer functionalization can be obtained, as required.

### Example 4 Preparation of a polymer matrix which includes the anti-microbial active drug component ciprofloxacin

A suspension in water (0.5 mL) of the polyglutamic acid polymer functionalized with cysteine, polyGlu(Cys)_{0,21}-OH, as obtained in example 1 (14.27 mg, approximately 19.5 kDa), is treated with an excess of NaBH₄ (approximately 15 mg) in order to reduce it and make it soluble (pH 9.3). The reduced solution is acidified with HCl to pH 1-2 in order to destroy the hydride residues and then added to a solution of ciprofloxacin hydrochloride (CFX.HCl, 3.02 mg, in borax buffer 0.5 ml pH 9-10). The resulting solution is diluted with additional borax buffer (to a final volume of 3.5 mL) and the pH is adjusted to 9.94 with NaOH 10 N (1-2 µL). The resulting solution is shaken constantly at room temperature for 90 h; at this point a suspension appears, produced by the polymer crosslinking due to oxidation.

In order to retrieve the polymer with the entrapped drug, the solution is acidified with HCl (to pH 1.8), whilst being shaken continuously, and cooled in a water/ice bath. This causes the drug/polymer matrix to precipitate. The precipitate and the solution are then placed in a concentration device (Ultrafree-15, Millipore^{®}), containing a MWCO 5000 Da membrane, and this enables the polymer (with the encapsulated drug) to be separated from the non-encapsulated drug which remains in solution by means of centrifugation. Measurement of the ciprofloxacin in the centrifugate (UV spectroscopy at λ= 272 nm) reveals that it constitutes 9% of the matrix's weight. A solid form of the drug/polymer matrix can be obtained by lyophilization of the solution.

### Example 5 Release kinetics of ciprofloxacin

A matrix formed by the polymer polyGlu(Cys)_{0.21}-OH and ciprofloxacin, 7.5% of its weight being the drug (1.5 mg of drug/polymer matrix), was suspended in an AcONH₄-buffered solution (1.5 ml, 25 mM, pH 7) of the enzyme endoproteinase Glu-C (300 µg, 0.20 µg/µl, BOEHRINGER). The mixture was held in suspension by means of magnetic stirring and kept at a constant temperature of 37 °C. In order to monitor both degradation of the polymer and release of the antibiotic, samples (150 µl) were taken at different times over several days. The samples were centrifuged with an ultrafiltration device containing an MWCO 5 kDa membrane (Ultrafree-0.5, Millipore^{®}, 3000 rpm for 45 min.), such that the ciprofloxacin released owing to enzymatic hydrolysis was present in the centrifuged solution. In order to establish the release profile of the ciprofloxacin, the centrifugates were analysed by UV-Vis spectroscopy (λ = 272 nm). The results of this analysis are presented in Figure 1, the graph showing µmol of ciprofloxacin (CFX) against time. For purposes of comparison the graph represents both an enzymatic proteolysis sample (M1) and that from a second control experiment without enzyme (B).

The graph shows that after 8 days approximately 28% of the drug had been released owing to enzymatic action.

### Example 6 Preparation of a polymer matrix including a peptide

In order to evaluate the entrapment capacity of peptide materials the same method as in example 5 was used to prepare a matrix including the peptide Ac-Thr-Tyr-Gln-Arg-Arg-Ser-Arg-Trp-Pro-Phe-Ser-Lys-Ala-Arg-Ser-CONH₂ (with a molecular weight of 1968.5), using polyGlu(Cys)_{0.05} of 17 kDa (functionalized with 5% of cysteine). Determination by UV spectroscopy at 280 nm revealed that the entrapment yield was 12% in weight.

### Example 7 Preparation and Use of a polymer matrix which includes human Growth Hormone

A solution of polyglutamic acid polymer, obtained by the procedure described in Example 1 and adapted to obtain a polymer with higher cysteine content, (polyGlu(Cys)0.4-OH, approximately 23 kDa, 6g) in NaOH 1M (18.6 ml) is added to lyophilized recombinant human growth hormone (hGH, 1g) with gentle shaking until a homogeneous solution is obtained (pH 6-7). DMSO (4.6 ml), the oxidation agent, is then added and after a gentle shaking the solution is left at room temperature for 12 h. A gel is obtained, which is washed with abundant water (2L) in a filter device of 0.22 µm (Durapore, Millipore®). After lyophilizing the remaining gel, a white powder (6.38 g) is obtained with a 4.5% w/w hGH content, determined by amino acid analysis.

### (a) Preliminary Pharmacokinetic Study In The Rat After Single Subcutaneous Administration-- Study Design

Three groups of five Sprague-Dawley CD (albino) male rats were administered Human Growth Hormone according to the following schedule:

| **Group** | **Dose route** | **Dose volume** | **Dose level** |
|---|---|---|---|
| 1 | Subcutaneous | 1 mL/kg | Depot alone |
| 2 | Subcutaneous | 1 mL/kg | Depot + 25 µg HGH/5 mg |
| 3 | Subcutaneous | 1 mL/kg | Depot + 250 µg HGH/5 mg |

Animals were in the age range 7 - 9 weeks and their bodyweights are given in Table 1.

### (b) Sample Collection

Following subcutaneous administration, blood samples (ca 0.5 mL) were taken from each rat at pre-dose, 0.5, 2, 4 and 8 hours post-dose.

All blood samples were collected into EDTA tubes from a caudal vein by venepunture, except the terminal blood sample, which was collected by cardiac puncture under isoflurane anaesthesia.

The blood samples were centrifuged and the separated plasma transferred to a clean tube. The blood cells were discarded.

Following collection of the terminal blood sample, the rats were killed by cervical dislocation and the liver, kidney, lungs, spleen, heart, brain and bone marrow removed. The plasma samples were stored at ca -80°C until taken for analysis. The carcasses were discarded.

### (c) Bioanalytical Methods

Plasma concentrations of Human Growth Hormone were assessed using a DPC (Los Angeles, California, USA) Radioimmunoassay kit on the Canberra Packard Cobra Gamma Counter, using kit manufacturer instructions. Standard calibration curve was obtained with 0.3-31 ng/ml HGH. Intra-assay coefficients of variation with the standard curve were 3% or less at each HGH concentration. Results of these experiments are summarized in Tables 1 and 2.

### (d) Results

Clinical Signs: No adverse reactions were observed following dose administration.

**TABLE 1**

| | **Animal weights and dose volumes** | | |
|---|---|---|---|
| **Group 1** | | | |
| | Animal No. | Weight (kg) | Volume (mL) |
| | 1 | 0.317 | 0.32 |
| | 2 | 0.321 | 0.32 |
| | 3 | 0.328 | 0.33 |
| | 4 | 0.318 | 0.33 |
| | 5 | 0.320 | 0.32 |
| | Mean | 0.321 | 0.32 |
| | sd | 0.004 | 0.01 |

| **Group 2** | | | |
|---|---|---|---|
| | Animal No. | Weight (kg) | Volume (mL) |
| | 6 | 0.316 | 0.32 |
| | 7 | 0.326 | 0.33 |
| | 8 | 0.332 | 0.33 |
| | 9 | 0.331 | 0.33 |
| | 10 | 0.337 | 0.34 |
| | Mean | 0.328 | 0.33 |
| | sd | 0.008 | 0.01 |

| **Group 3** | | | |
|---|---|---|---|
| | Animal No. | Weight (kg) | Volume (mL) |
| | 11 | 0.316 | 0.32 |
| | 12 | 0.315 | 0.32 |
| | 13 | 0.316 | 0.32 |
| | 14 | 0.341 | 0.34 |
| | 15 | 0.335 | 0.34 |
| | Mean | 0.325 | 0.33 |
| | sd | 0.012 | 0.01 |

| | | | |
|---|---|---|---|
| sd = Standard deviation Age range = 7 - 9 weeks | | | |

**TABLE 2**

| **Plasma concentrations of Human Growth Hormone in rats following subcutaneous administration of depot alone (Group 1), depot + 25 µg HGH (Group 2) and depot + 250 µg HGH (Group 3)** | | | | | | | |
|---|---|---|---|---|---|---|---|
| **Group 1** | | | | | | | |
| Time (hours) | Concentration (ng/mL) | | | | | | |
| | 1 | 2 | 3 | 4 | 5 | Mean | sd |
| 0 | 0.33 | 0.49 | 0.52 | 0.14 | 0.48 | 0.39 | 0.16 |
| 0.5 | NR | 1.31 | 0.58 | 0.62 | 0.94 | 0.86 | 0.34 |
| 2 | 0.55 | 1.12 | 0.60 | 0.56 | 1.16 | 0.80 | 0.31 |
| 4 | 0.43 | 0.63 | 0.65 | 0.60 | 0.71 | 0.60 | 0.11 |
| 8 | 0.58 | 1.67 | 1.50 | 1.23 | 1.60 | 1.32 | 0.44 |

| **Group 2** | | | | | | | |
|---|---|---|---|---|---|---|---|
| Time (hours) | Concentration (ng/mL) | | | | | | |
| | 6 | 7 | 8 | 9 | 10 | Mean | sd |
| 0 | 0.65 | 0.67 | 0.96 | 0.56 | 0.37 | 0.64 | 0.21 |
| 0.5 | 1.52 | 2.38 | 2.13 | 2.71 | 2.11 | 2.17 | 0.44 |
| 2 | 3.11 | 3.04 | 2.98 | 5.27 | 3.31 | 3.54 | 0.97 |
| 4 | 1.56 | 1.95 | 1.90 | 2.72 | 1.96 | 2.02 | 0.43 |
| 8 | 0.88 | 1.62 | 1.13 | 1.25 | 1.01 | 1.18 | 0.28 |

| **Group 3** | | | | | | | |
|---|---|---|---|---|---|---|---|
| Time (hours) | Concentration (ng/mL) | | | | | | |
| | 11 | 12 | 13 | 14 | 15 | Mean | sd |
| 0 | 0.67 | 0.73 | 0.61 | 0.60 | 0.61 | 0.64 | 0.06 |
| 0.5 | 8.50 | 17.53 | 17.64 | 6.20 | 13.77 | 12.73 | 5.21 |
| 2 | 8.20 | 23.91 | 27.39 | 27.47 | 15.96 | 20.59 | 8.36 |
| 4 | 3.02 | 13.66 | 19.76 | 18.21 | 10.34 | 13.00 | 6.71 |
| 8 | 1.16 | 4.59 | 8.40 | 5.04 | 3.06 | 4.45 | 2.68 |

| | | | | | | | |
|---|---|---|---|---|---|---|---|
| NR No result sd Standard deviation | | | | | | | |

### (e) Conclusions

This example and its results indicate that the present HGH depot formulation can provide concentration- and time- dependent release of HGH after single subcutaneous injection in rats, in the absence of overt clinical signs. At the highest HGH concentration (Group 3), plasma HGH levels were elevated from 30 minutes to 8 hours after single subcutaneous administration. This example in vivo therefore supports the feasibility of using this embodiment of the invention to obtain reliable and relatively long-lasting potentially therapeutic plasma levels of HGH.

It should be understood that the Examples, while indicating preferred embodiments of the invention, are given by way of illustration only. From the above discussion and the Examples, one skilled in the art can ascertain the essential characteristics of this invention, and without departing from the spirit and scope thereof, can make various changes and modifications of the invention to adapt it to various usages and conditions.

## Claims

1. A polypeptide comprising between about 1% and about 75% of monomeric units having one or more of the following formulas (I), (II) or (III): 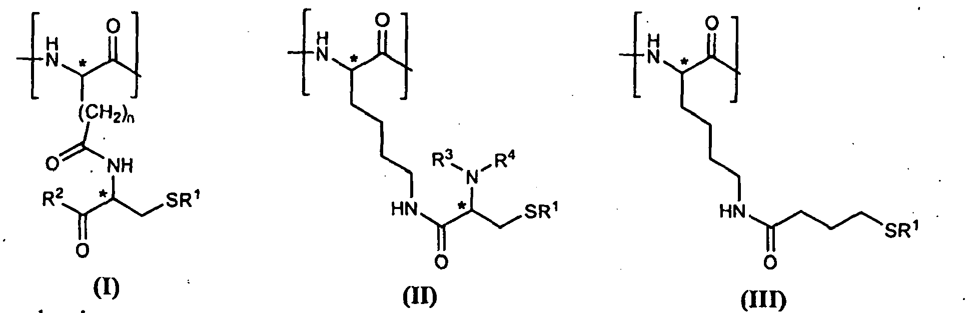 wherein:
- n may be 1 or 2;
- R¹ may be H or a thiol protective group;
- R² may be a hydroxyl group (OH), a carboxylic acid protective group, or an - NR⁵R⁶ group in which R⁵ and R⁶ may be selected from the group consisting of H or a C₁-C₇ alkyl group, linear or branched;
- R³ and R⁴ may be selected from the group consisting of H, a C₁-C₇ alkyl group, linear or branched, or an amine protective group
wherein the chiral centers marked with an asterisk may have an *R*, *S* or *RS* configuration.

2. A polypeptide according to claim 1 therein the monomer units comprise Formula (I).

3. A polypeptide according to claim 1 wherein the monomer units comprise Formula (II).

4. A polypeptide according to claim 1 wherein the monomer units comprise Formula (III).

5. A polypeptide according to claim 1, comprising between about 5% and about 50% of the monomeric units having one or more of the formulas (I), (II) or (III).

6. A polypeptide according to claim 1, wherein the polymer molecular weight is between about 5,000 and about 200,000.

7. A polypeptide according to claim 6 wherein the polymer molecular weight is between about 10,000 and about 100,000.

8. A polypeptide according to claim 1, in which at least about 50% of the monomeric units have one or more of the formulas (IV) or (V): 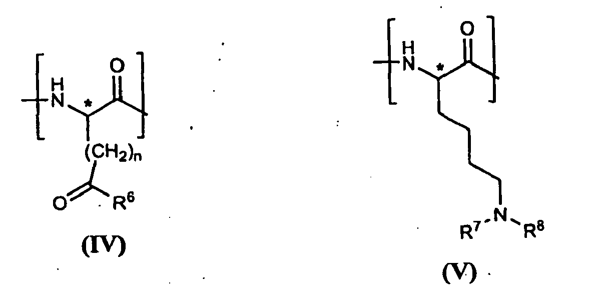 wherein:
- n can be 1 or 2;
- R⁶ can be a hydroxyl group (OH), a carboxylic acid protective group, an -NR³R⁴ group in which R³ and R⁴ are as stated above, or a residue of the formula: 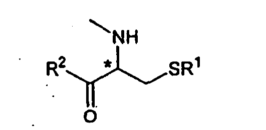 where R¹ and R² are as stated above;
- R⁷ and R⁸ can be H, a C₁-C₇ alkyl group, linear or branched, or a primary amine protective group, and in which case either R⁷ or R⁸ is H, or a residue of the formula: 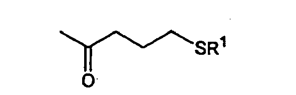 in which case either R⁷ or R⁸ is H and R¹ is as stated above, or a residue of the formula: 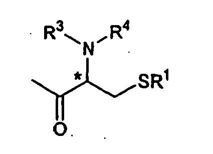 in which case either R⁷ or R⁸ is H and R¹, R³ and R⁴ are as stated above,
wherein the chiral centres marked with an asterisk may have an *R*, *S* or *RS* configuration.

9. A polypeptide according to claim 8 wherein the monomeric units comprise Formula (IV).

10. A polypeptide according to claim 8 wherein the monomeric units comprise Formula (V).

11. The polypeptide according to claim 8, wherein the polypeptide can contain other monomeric units selected from among other natural or non-natural amino acids and their protected derivatives.

12. The polypeptide according to claim 8 in which at least about 80% of the monomeric units have either formula (IV) or (V).

13. The polypeptide according to claim 8 wherein between about 5% and about 30% of the monomeric units contain SR¹ groups.

14. The polypeptide according to claim 8 wherein the monomeric units are selected from among those of formula (IV), those of formula (V), or L-alanine and D-alanine residues.

15. The polypeptide according to claim 8 comprising monomeric units of formula (IV) or (V) in which R¹ is H.

16. The polypeptide according to claim 8 comprising monomeric units of formula (IV) in which R⁶ is OH or a cysteine residue, or those of formula (V) in which R⁷ and R⁸ are H or R⁷ is H and R⁸ is a residue of either cysteine or 4-mercaptobutyroyl.

17. The polypeptide according to claim 1 comprising monomeric units of residues of L-glutamic acid and L-glutamic acid (L-cysteine) with the formulas

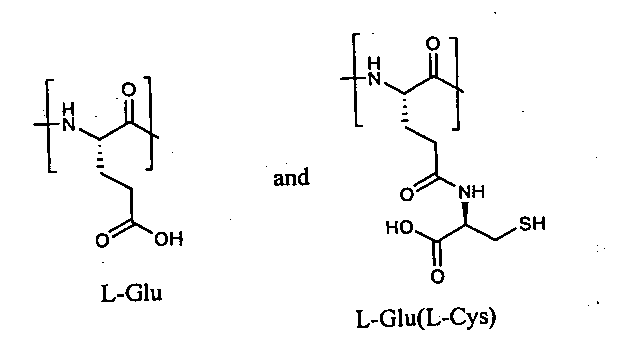

18. The polypeptide according to claim 17 further comprising monomeric units of L-alanine residues.

19. The polypeptide according to claim 1 comprising monomeric units of residues of L-lysine and N'-(4-mercaptobutyroyl)-L-lysine with the formulas:

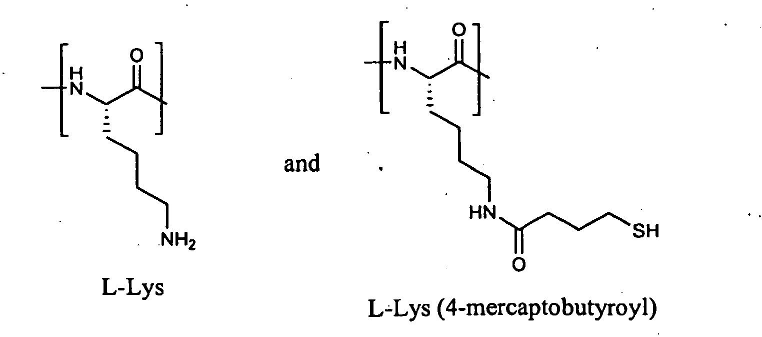

20. The polypeptide according to claim 19 further comprising monomeric units of L-alanine residues.

21. A method for preparing the polypeptides described in claim 1, comprising providing a precursor polypeptide in which at least about 50% of the monomeric units have the formula (VI) or (VII): 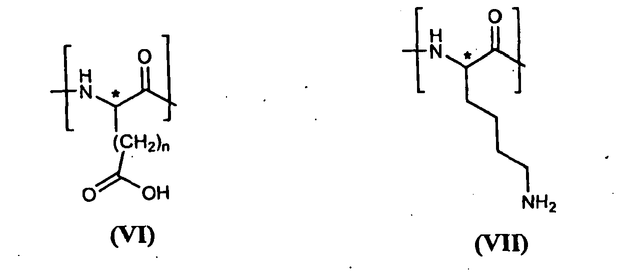 or their precursors in which either the carboxylic acid group or the free primary amine group is protected by protective groups, and
- the precursor polypeptide containing monomeric units of formula (VI) is reacted with cysteine or its protected derivatives with the formula 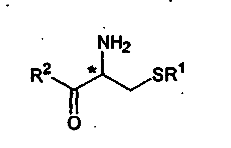 in which R¹ and R² are as stated above; or alternatively,
- the precursor polypeptide containing monomeric units of formula (VII) is reacted with cysteine or its protected derivatives with the formula 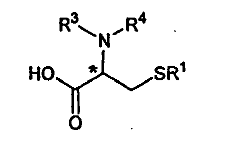 or with 4-mercaptobutyric acid or its activated derivatives.

22. A method for preparing a polymer matrix containing at least one drug, comprising oxidizing in alkaline aqueous medium a polypeptide according to claim 1 in the presence of the at least one drug, and separating the resulting solution.

23. The polymer matrix containing a drug which is obtained using the method according to claim 22.

24. A polymer matrix comprising a crosslinked polypeptide of claim 1 or claim 8.

25. A polymer matrix comprising a crosslinked polypeptide of claim 24 and at least one drug.

26. The polymer matrix according to claim 23 or 25 wherein the drug is a peptide or a protein.

27. The polymer matrix according to claim 26 comprising a drug component comprising one or more of the following: T-20 peptide; interferon or other interleucines; gonadotropin-releasing hormone(GnRH) analogues, such as leuprolide, goserelin, nafarelin or triptorelin; human growth hormone; follicle-stimulating hormone (FSH), or luteinizing hormone (LH).

28. The polymer matrix according to claim 23 or 25 wherein the drug component comprises paclitaxel, doxorrubicin or ciprofloxacin.

29. The polymer matrix according to claim 23 or claim 25 wherein the drug component is human growth hormone.

30. A pharmaceutical composition comprising a polymer matrix containing a drug according to claim 23 or 25 and a pharmaceutically acceptable excipient, extender or carrier.

31. A polypeptide according to claim 1 wherein the polypeptide can contain other monomeric units selected from among other natural or non-natural amino acids and their protected derivatives.

## Patentansprüche

1. Polypeptid, umfassend etwa 1 % bis etwa 75 % Monomereinheiten mit einer der folgenden Formeln (I), (II) oder (III): 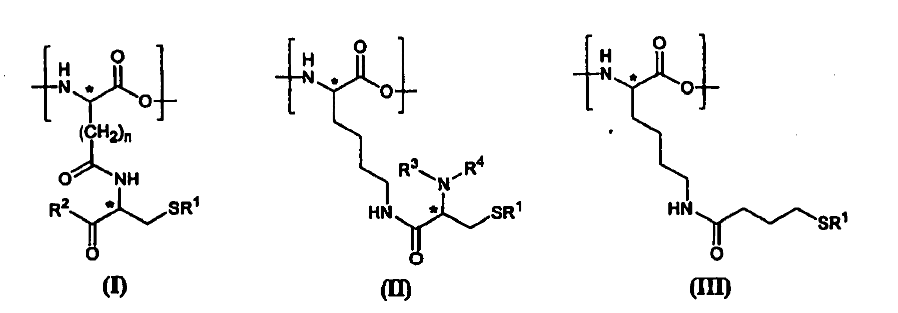 worin:
- n für 1 oder 2 steht;
- R¹ für H oder eine Thiolschutzgruppe steht;
- R² für eine Hydroxygruppe (OH), eine Carbonsäureschutzgruppe oder eine
- NR⁵R⁶-Gruppe steht, worin R⁵ und R⁶ ausgewählt sind unter H oder einer geradkettigen oder verzweigten C₁-C₇-Alkylgruppe;
- R³ und R⁴ ausgewählt sind unter H, einer geradkettigen oder verzweigten C₁-C₇-Alkylgruppe oder einer Aminschutzgruppe,
wobei die mit einem Stern **gekennzeichneten** chiralen Zentren eine *R*, *S* oder *RS-*Konfiguration besitzen.

2. Polypeptid nach Anspruch 1, worin die Monomereinheiten Formel (I) umfassen.

3. Polypeptid nach Anspruch 1, worin die Monomereinheiten Formel (II) umfassen.

4. Polypeptid nach Anspruch 1, worin die Monomereinheiten Formel (III) umfassen.

5. Polypeptid nach Anspruch 1, umfassend etwa 5 % bis etwa 50 % von Monomereinheiten mit einer oder mehreren der Formeln (I), (II) oder (III).

6. Polypeptid nach Anspruch 1, worin das Molekulargewicht des Polymers etwa 5.000 bis etwa 200.000 beträgt.

7. Polypeptid nach Anspruch 6, worin das Molekulargewicht des Polymers etwa 10.000 bis etwa 100.000 beträgt.

8. Polypeptid nach Anspruch 1, worin wenigstens etwa 50 % der Monomereinheiten eine oder mehrere der Formeln (IV) oder (V) aufweisen: 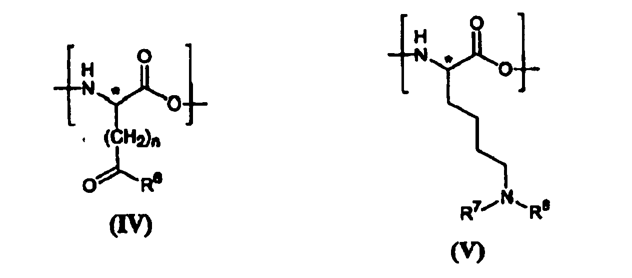 worin
- n für 1 oder 2 steht;
- R⁶ eine Hydroxylgruppe (OH), eine Carbonsäureschutzgruppe, eine -NR³R⁴-Gruppe, worin R³ und R⁴ die oben angegebenen Bedeutungen besitzen, oder einen Rest der Formel: 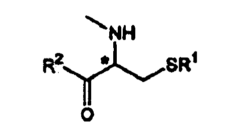 bedeutet, worin R¹ und R² die oben angegebenen Bedeutungen besitzen;
- R⁷ und R⁸ für H, eine geradkettige oder verzweigte C₁-C₇-Alkylgruppe oder eine primäre Amin-Schutzgruppe stehen, in welchem Fall entweder R⁷ oder R⁸ für H oder einen Rest der Formel: 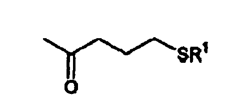 steht, in welchem Fall R⁷ oder R⁸ für H steht und R¹ die oben angegebenen Bedeutungen besitzt, oder für einen Rest der Formel: 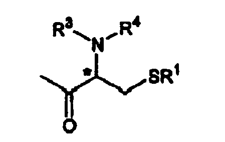 steht, in welchem Fall entweder R⁷ oder R⁸ für H steht und R¹, R³ und R⁴ die oben angegebenen Bedeutungen besitzen,
wobei die mit einem Stern **gekennzeichneten** chiralen Zentren eine *R*, *S* oder *RS-*Konfiguration besitzen.

9. Polypeptid nach Anspruch 8, worin die Monomereinheiten Formel (IV) umfassen.

10. Polypeptid nach Anspruch 8, worin die Monomereinheiten Formel (V) umfassen.

11. Polypeptid nach Anspruch 8, worin das Polypeptid andere Monomereinheiten enthalten kann, die ausgewählt sind unter natürlichen oder nicht-natürlichen Aminosäuren und den geschützten Derivaten davon.

12. Polypeptid nach Anspruch 8, worin wenigstens etwa 80 % der Monomereinheiten entweder Formel (IV) oder Formel (V) aufweisen.

13. Polypeptid nach Anspruch 8, worin etwa 5 % bis etwa 30 % der Monomereinheiten SR¹-Gruppen aufweisen.

14. Polypeptid nach Anspruch 8, worin die Monomereinheiten ausgewählt sind unter denjenigen der Formel (IV), denjenigen der Formel (V), oder L-Alanin- oder D-Alanin-Resten.

15. Polypeptid nach Anspruch 8, umfassend Monomereinheiten der Formel (IV) oder (V), worin R¹ für H steht.

16. Polypeptid nach Anspruch 8, umfassend Monomereinheiten der Formel (IV), worin R⁶ für OH oder einen Cysteinrest steht, oder solchen der Formel (V), worin R⁷ und R⁸ für H stehen oder R⁷ für H steht und R⁸ für einen Cystein- oder 4-Mercaptobutyroyl-Rest steht.

17. Polypeptid nach Anspruch 1, umfassend Monomereinheiten der Reste von L-Glutaminsäure und L-Glutaminsäure(L-Cystein) mit den Formeln:

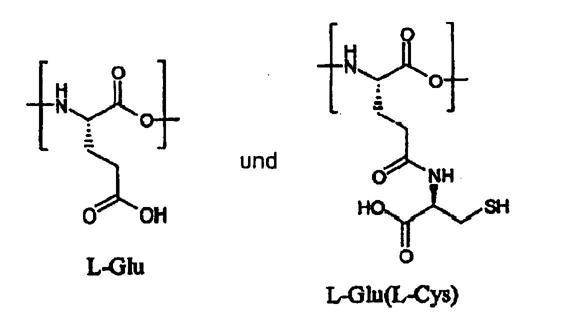

18. Polypeptid nach Anspruch 17, zusätzlich umfassend Monomereinheiten von L-Alanin-Resten.

19. Polypeptid nach Anspruch 1, umfassend Monomereinheiten der Reste von L-Lysin und N'-(4-Mercaptobutyroyl)-L-lysin) der Formeln:

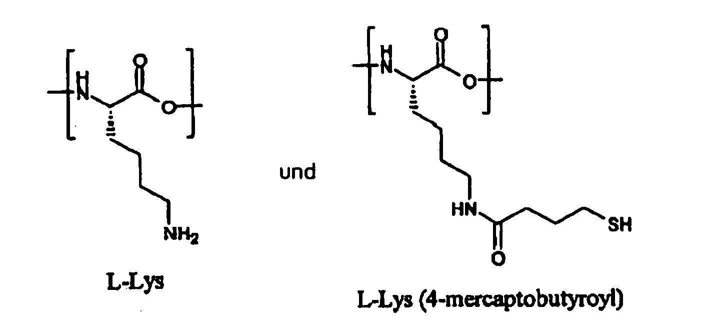

20. Polypeptid nach Anspruch 19, zusätzlich umfassend Monomereinheiten von L-Alanin-Resten.

21. Verfahren zur Herstellung des Polypeptids nach Anspruch 1, umfassend die Bereitstellung eines Vorläufer-Polypeptids, in welchem wenigstens 50 % der Monomereinheiten die Formel (VI) oder (VII) aufweisen: 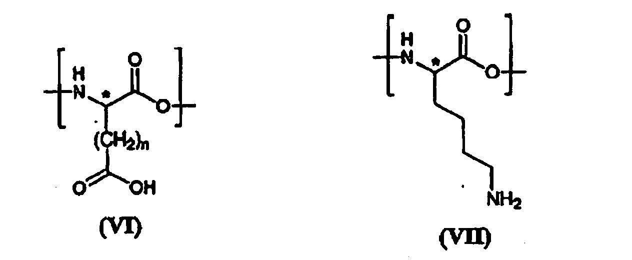 oder deren Vorläufer, worin entweder die Carbonsäuregruppe oder die freie primäre Amingruppe durch Schutzgruppen geschützt ist, und wobei man
- das Vorläufer-Polypeptid, enthaltend Monomereinheiten der Formel (VI) mit Cystein oder dessen geschützten Derivaten der Formel 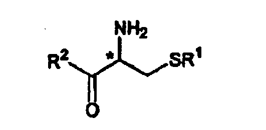 worin R¹ und R² die oben angegebenen Bedeutungen besitzen, umsetzt oder alternativ dazu
- das Vorläufer-Polypeptid, enthaltend Monomereinheiten der Formel (VII) mit Cystein oder geschützten Derivaten davon, der Formel 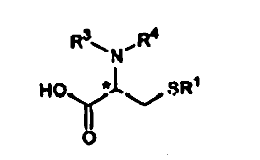 oder mit 4-Mercaptobuttersäure oder Derivaten davon umsetzt.

22. Verfahren zur Herstellung einer Polymermatrix, enthaltend wenigstens einen Wirkstoff, wobei man in alkalischem wässrigem Medium ein Polypeptid nach Anspruch 1 in Gegenwart wenigstens eines Wirkstoffs oxidiert und die resultierende Lösung abtrennt.

23. Polymermatrix, enthaltend einen Wirkstoff, hergestellt unter Verwendung des Verfahrens nach Anspruch 22.

24. Polymermatrix, umfassend ein vernetztes Polypeptid nach Anspruch 1 oder Anspruch 8.

25. Polymermatrix, umfassend ein vernetztes Polypeptid nach Anspruch 24 und wenigstens einen Wirkstoff.

26. Polymermatrix nach einem der Ansprüche 23 oder 25, worin der Wirkstoff ein Peptid oder Protein ist.

27. Polymermatrix nach Anspruch 26, umfassend eine Wirkstoffkomponente, umfassend einen oder mehrere der folgenden Verbindungen: T-20-Peptid; Interferon oder andere Interleukine; Gonadotropin-freisetzendes Hormon (GnRH) Analoga, wie Leuprolid, Goserelin, Nafarelin oder Triptorelin; menschliches Wachstumshormon; Follikelstimulierendes Hormon (FSH) oder luteinisierendes Hormon (LH).

28. Polymermatrix nach Anspruch 23 oder 25, worin die Wirkstoffkomponente Paclitaxel, Doxorubicin oder Ciprofloxacin umfasst.

29. Polymermatrix nach Anspruch 23 oder Anspruch 25, worin die Wirkstoffkomponente menschliches Wachstumshormon ist.

30. Pharmazeutische Zusammensetzung, umfassend eine Polymermatrix, enthaltend einen Wirkstoff nach Anspruch 23 oder 25 und einen pharmazeutisch verträglichen Exzipienten, Extender oder Träger.

31. Polypeptid nach Anspruch 1, worin das Polypeptid andere Monomereinheiten, ausgewählt unter natürlichen oder nicht-natürlichen Aminosäuren und geschützten Derivaten davon enthält.

## Revendications

1. Polypeptide comprenant entre environ 1 % et environ 75 % de motifs monomères ayant une ou plusieurs des formules (I), (II) ou (III) suivantes : 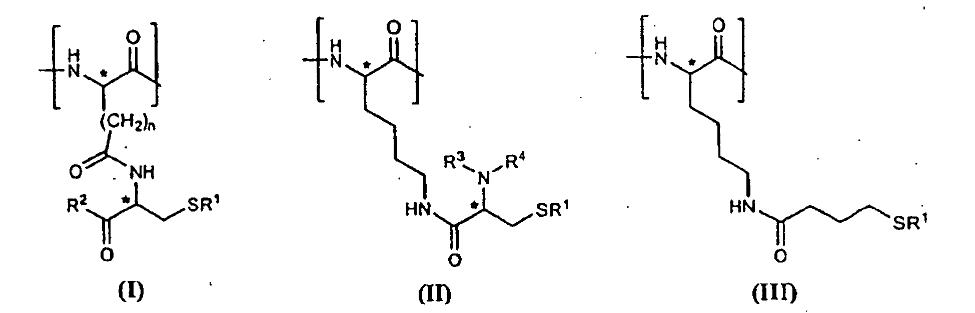 dans lesquelles :
- n peut être 1 ou 2 ;
- R¹ peut être H ou un groupe de protection d'un thiol ;
- R² peut être un groupe hydroxyle (OH), un groupe de protection d'un acide carboxylique ou un groupe-NR⁵R⁶ dans lequel R⁵ et R⁶ peuvent être choisis dans le groupe consistant en H ou en un groupe alcoyle, linéaire ou ramifié, ayant de 1 à 7 atomes de carbone ;
- R³ et R⁴ peuvent être choisis dans le groupe consistant en H, un groupe alcoyle, linéaire ou ramifié, ayant de 1 à 7 atomes de carbone ou un groupe de protection d'une amine
dans lesquelles les centres chiraux repérés par un astérisque peuvent avoir une configuration *R*, *S* ou *RS.*

2. Polypeptide suivant la revendication 1, dans lequel les motifs monomères comportent la formule (I).

3. Polypeptide suivant la revendication 1, dans lequel les motifs monomères comportent la formule (II).

4. Polypeptide suivant la revendication 1, dans lequel les motifs monomères comportent la formule (III).

5. Polypeptide suivant la revendication 1, comprenant entre environ 5 % et environ 50 % des motifs monomères ayant une ou plusieurs des formules (I), (II) ou (III).

6. Polypeptide suivant la revendication 1, dans lequel la masse moléculaire du polymère est comprise entre environ 5 000 et environ 200 000.

7. Polypeptide suivant la revendication 6, dans lequel la masse moléculaire du polymère est comprise entre environ 10 000 et environ 100 000.

8. Polypeptide suivant la revendication 1, dans lequel au moins environ 50 % des motifs monomères ont l'une ou plusieurs des formules (IV) ou (V) : 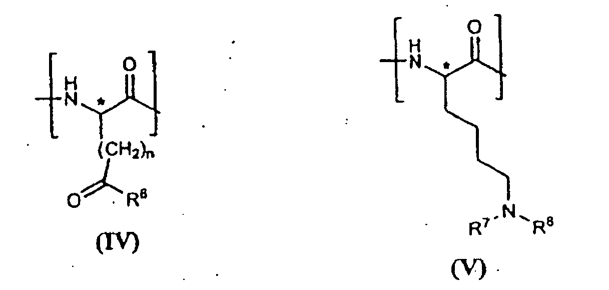 dans lesquelles :
- n peut être 1 ou 2 ;
- R⁶ peut être un groupe hydroxyle (OH), un groupe de protection d'un acide carboxylique, un groupe-NR³R⁴ dans lequel R³ et R⁴ sont tels qu'indiqués ci-dessus ou un radical de formule : 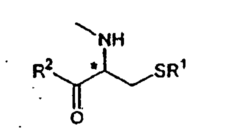 dans laquelle R¹ et R² sont tels qu'indiqués ci-dessus ;
- R⁷ et R⁸ peuvent être H, un groupe alcoyle, linéaire ou ramifié, ayant de 1 à 7 atomes de carbone ou un groupe de protection d'une amine primaire et, auquel cas, soit R⁷, soit R⁸ est H, ou un radical de formule : 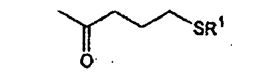
auquel cas soit R⁷ soit R⁸ est H et R¹ est tel qu'indiqué ci-dessus, ou un radical de formule : 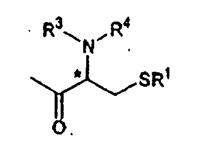 auquel cas soit R⁷, soit R⁸ est H et R¹, R³ et R⁴ sont tels qu'indiqués ci-dessus,
dans lesquelles les centres chiraux repérés par un astérisque peuvent avoir une configuration *R*, *S* ou *RS.*

9. Polypeptide suivant la revendication 8, dans lequel les motifs monomères comportent la formule (IV).

10. Polypeptide suivant la revendication 8, dans lequel les motifs monomères comportent la formule (V).

11. Polypeptide suivant la revendication 8, dans lequel le polypeptide peut contenir d'autres motifs monomères choisis parmi d'autres acides aminés naturels ou qui ne le sont pas et leurs dérivés protégés.

12. Polypeptide suivant la revendication 8, dans lequel au moins 80 % environ des motifs monomères ont la formule (IV) ou la formule (V).

13. Polypeptide suivant la revendication 8, dans lequel entre environ 5 % et environ 30 % des motifs monomères contiennent des groupes SR¹.

14. Polypeptide suivant la revendication 8, dans lequel les motifs monomères sont choisis par ceux de formule (IV), ceux de formule (V) ou des radicaux L-alanine et D-alanine.

15. Polypeptide suivant la revendication 8, comprenant des motifs monomères de formule (IV) ou (V) dans lesquelles R¹ est H.

16. Polypeptide suivant la revendication 8, comprenant des motifs monomères de formule (IV) dans laquelle R⁶ et OH ou un radical cystéine ou ceux de formule (V) dans laquelle R⁷ et R⁸ sont H ou R⁷ est H et R⁸ est un radical cystéine ou 4-mercaptobutyroyle.

17. Polypeptide suivant la revendication 1, comprenant des motifs monomères de radicaux d'acide L-glutamique et d'acide L-glutamique(L-cystéine) de formules

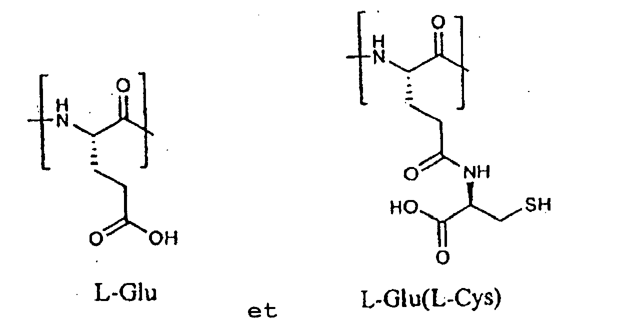

18. Polypeptide suivant la revendication 17, comprenant, en outre, des motifs monomères de radicaux d'acide L-alanine.

19. polypeptide suivant la revendication 1, comprenant des motifs monomères de radicaux de L-lysine et de N'-(4-mercaptobutyroyl)-L-lysine de formules :

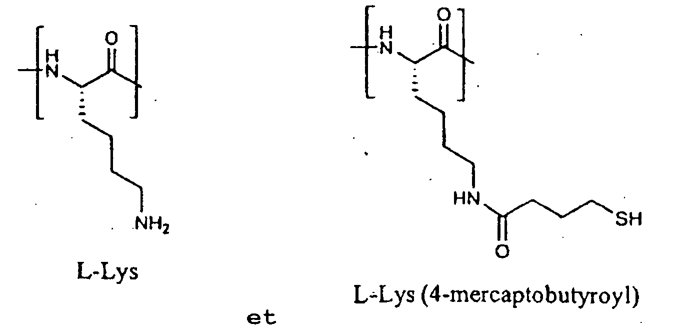

20. Polypeptide suivant la revendication 19, comprenant, en outre, des motifs monomères de radicaux L-alanine.

21. Procédé de préparation des polypeptides décrits à la revendication 1, dans lequel on prévoit un polypeptide précurseur, dans lequel au moins environ 50 % des motifs monomères a la forme (VI) ou (VII) : 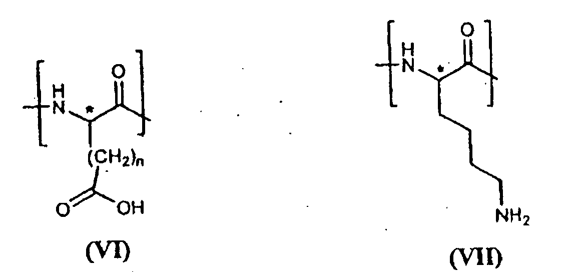 ou leurs précurseurs dans lesquels soit le groupe acide carboxylique, soit le groupe amine primaire libre est protégé par des groupes de protection,
- on fait réagir le polypeptide précurseur contenant des motifs monomères de formule (VI) sur de la cystéine ou sur ses dérivés protégés de formule 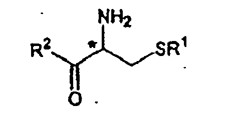 dans laquelle R¹ et R² sont tels qu'indiqués ci-dessus ; ou en variante,
- on fait réagir le polypeptide précurseur contenant des motifs monomères de formule (VII) sur de la cystéine ou sur ses dérivés protégés de formule 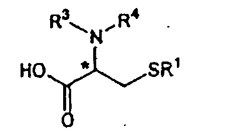
ou sur de l'acide 4-mercaptobutyrique ou sur ses dérivés activés.

22. Procédé de préparation d'une matrice polymère contenant au moins un médicament, dans lequel on oxyde dans un milieu alcalin aqueux un polypeptide suivant la revendication 1 en la présence du au moins un médicament et on sépare la solution obtenue.

23. Matrice polymère contenant un médicament qui est obtenu en utilisant le procédé suivant la revendication 22.

24. Matrice polymère comprenant un polypeptide réticulé suivant la revendication 1 ou suivant la revendication 8.

25. Matrice polymère comprenant un polypeptide réticulé suivant la revendication 24 et au moins un médicament.

26. Matrice polymère suivant la revendication 23 ou 25, dans laquelle le médicament est un peptide ou une protéine.

27. Matrice polymère suivant la revendication 26, comprenant un constituant médicamenteux comprenant un ou plusieurs des suivants : peptide T-20, interféron ou d'autres interleucines ; analogues à l'hormone de libération de la gonadotropine (GnRH), tels que leuprolide, goséréline, nafaréline ou triptoréline ; hormone de croissance humaine ; hormone de stimulation des follicules (FSH) ou hormone lutéinisante (LH).

28. Matrice polymère suivant la revendication 23 ou 25, dans laquelle le constituant médicamenteux comprend du paclitaxel, de la doxorubicine ou de la ciprofloxacine.

29. Matrice polymère suivant la revendication 23 ou 25, dans laquelle le constituant médicamenteux est une hormone de croissance humaine.

30. Composition pharmaceutique comprenant une matrice polymère contenant les médicaments suivant la revendication 23 ou 25 et un excipient, un diluant ou un véhicule acceptable pharmaceutiquement.

31. polypeptide suivant la revendication 1, dans lequel le polypeptide peut contenir d'autres motifs monomères choisis parmi d'autres acides aminés naturels ou qui ne le sont pas et leurs dérivés protégés.
